(19) Europäisches Patentamt
European Patent Office
Office européen des brevets

(11) Veröffentlichungsnummer: **0 135 105**
**B1**

(12) # EUROPÄISCHE PATENTSCHRIFT

(45) Veröffentlichungstag der Patentschrift: **10.08.88**

(51) Int. Cl.⁴: **A 01 N 43/50**, A 01 N 43/34, C 07 D 403/06

(21) Anmeldenummer: **84109310.7**

(22) Anmeldetag: **06.08.84**

(54) **Mittel zur Bekämpfung von Insekten und Vertretern der Ordnung Akarina, welche als aktive Komponente 2-(1-Indolinyl-methyl)-imidazoline oder deren Salzen enthalten.**

(30) Priorität: **09.08.83 CH 4334/83**
**11.07.84 CH 3362/84**

(43) Veröffentlichungstag der Anmeldung: **27.03.85 Patentblatt 85/13**

(45) Bekanntmachung des Hinweises auf die Patenterteilung: **10.08.88 Patentblatt 88/32**

(84) Benannte Vertragsstaaten: **BE CH DE FR IT LI NL**

(56) Entgegenhaltungen:
**DE-A-1 670 143**
**FR-A-1 167 012**
**US-A-3 404 156**

(73) Patentinhaber: **CIBA- GEIGY AG, Klybeckstrasse 141, CH- 4002 Basel (CH)**

(72) Erfinder: **Burckhard, Urs, Dr., Rittergasse 29, CH-4051 Basel (CH)**

(74) Vertreter: **Zumstein, Fritz, Dr., Bräuhausstrasse 4, D-8000 München 2 (DE)**

**EP 0 135 105 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue 2-(1-Indolinyl-methyl)-imidazoline, Mittel zur Bekämpfung von Insekten und Vertretern der Ordnung Akarina, welche als aktive Komponente diese neuen 2-(1-Indolinyl-methyl)-imidazoline oder deren Salze enthalten, ihre Verwendung, sowie ein Verfahren zu ihrer Herstellung.

In der DE-A-1 670 143 ist die Verbindung der Formel

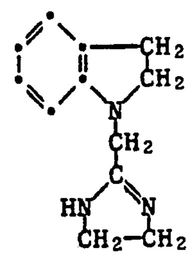

als "Vasoconstrictor" beschrieben.

Ziel der vorliegenden Erfindung war die Bereitstellung neuer insektizider Wirkstoffe.

Es wurde gefunden, daß die neuen Verbindungen der Formel I

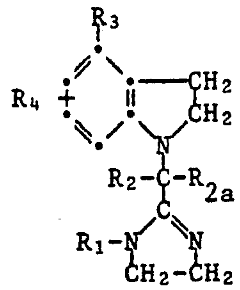

(I)

oder deren Salze,
worin,

$R_1$ Wasserstoff oder $C_1$-$C_{12}$-Alkyl,
$R_2$ und $R_{2a}$ unabhängig voneinander Wasserstoff, $C_1$-$C_4$-Alkyl oder zusammen $C_2$-$C_4$-Alkylen,
$R_3$ Wasserstoff, Halogen, $C_1$-$C_3$-Alkoxy oder gegebenenfalls durch Halogen substituiertes $C_1$-$C_4$-Alkyl und
$R_4$ Halogen, $C_1$-$C_3$-Alkoxy oder gegebenenfalls durch Halogen substituiertes $C_1$-$C_4$-Alkyl oder
$R_3$ Halogen, $C_1$-$C_3$-Alkoxy oder gegebenenfalls durch Halogen substituiertes $C_1$-$C_4$-Alkyl und
$R_4$ Wasserstoff, Halogen, $C_1$-$C_3$-Alkoxy oder gegebenenfalls durch Halogen substituiertes $C_1$-$C_4$-Alkyl

bedeuten, wertvolle insektizide Eigenschaften haben.

In den Verbindungen der Formel I ist unter Halogen Fluor, Chlor, Brom oder Jod, insbesondere aber Chlor oder Fluor zu verstehen.

Die bei $R_1$ bis $R_4$ stehenden Alkyl- oder Alkoxygruppen können geradkettig oder verzweigt sein.

Beispiele von Alkoxy- und gegebenenfalls durch Halogen substituierten Alkylgruppen bei $R_1$ bis $R_4$ sind u.a.: Methyl, Methoxy, Trifluormethyl, Äthyl, Äthoxy, Propyl, Propoxy, Isopropyl, n-, i-, sek.-, tert.-Butyl, n-Pentyl, n-Hexyl, n-Heptyl, n-Dodecyl und deren Isomere.

Unter $C_2$-$C_4$-Alkylen sind u.a. Äthylen oder Butylen zu verstehen.

Für die Salzbildung kommen anorganische Säuren wie beispielsweise HCl, $H_2SO_4$, HBr und $H_3PO_4$ und organische Säuren beispielsweise gesättigte und ungesättigte Mono-, Di- und Tricarbonsäuren wie z. B. Ameisensäure, Essigsäure, Trifluoressigsäure, Oxalsäure, Phthalsäure, Bernsteinsäure, Benzoesäure, p-Methylphenylsulfosäure und Zitronensäure in Betracht.

Bevorzugt sind Verbindungen der Formel I, worin
$R_1$ und $R_{2a}$ Wasserstoff,
$R_2$ Wasserstoff oder $C_1$-$C_4$-Alkyl,
$R_3$ Halogen, $C_1$-$C_3$-Alkoxy oder gegebenenfalls durch Fluor substituiertes $C_1$-$C_4$-Alkyl und
$R_4$ Wasserstoff, Halogen oder $C_1$-$C_4$-Alkyl bedeuten.

Insbesondere bevorzugt sind Verbindungen der Formel I;
worin $R_1$, $R_2$, $R_{2a}$ und $R_4$ Wasserstoff und
$R_3$ Halogen, $C_1$-$C_3$-Alkoxy oder gegebenenfalls durch Fluor substituiertes $C_1$-$C_4$-Alkyl bedeuten.

Die Verbindungen der Formel I werden nach an sich bekannten Methoden z. B. wie folgt hergestellt.

1)

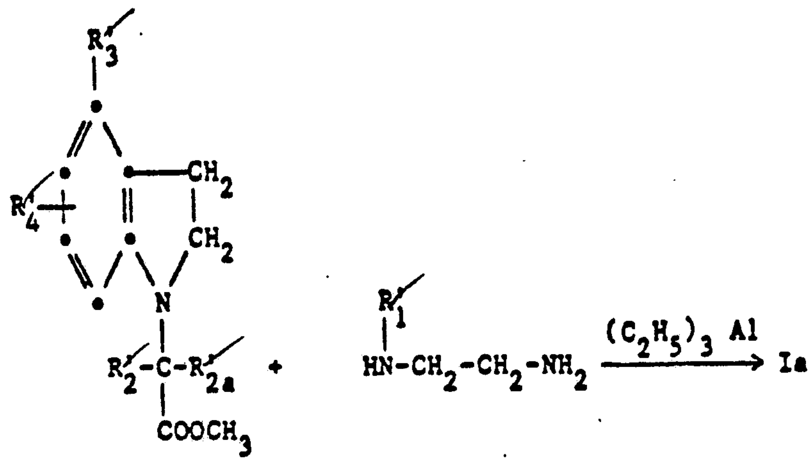


(II) (III)

2)

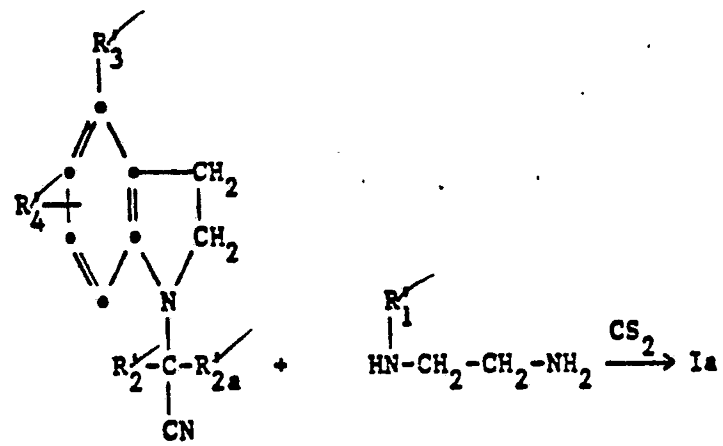


(IV) (III)

In den Formeln II, III und IV haben $R_1$ bis $R_4$ die für die Formel I angegebene Bedeutung.

Die Verfahren werden bei normalem Druck, bei einer Temperatur von -25 bis 150° C, vorzugsweise bei 50 bis 120° C und gegebenenfalls in einem Lösungs- oder Verdünnungsmittel durchgeführt.

Als Lösungs- oder Verdünnungsmittel eignen sich z. B. Äther und ätherartige Verbindungen wie Diäthyläther, Diisopropyläther, Dioxan und Tetrahydrofuran; aromatische Kohlenwasserstoffe wie Benzol, Toluol und Xylole; und halogenierte Kohlenwasserstoffe.

Die Ausgangsstoffe der Formeln II, III und IV sind bekannt und können nach bekannten Methoden hergestellt werden.

Die Verbindungen der Formel I eignen sich zur Bekämpfung von Schädlingen an Tieren und Pflanzen. Therapeutische Behandlungen sind hier jedoch ausgeschlossen.

Insbesondere eignen sich die Verbindungen der Formel I zur Bekämpfung von Insekten, z. B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Akarina.

Es ist hervorzuheben, daß die erfindungsgemässen Verbindungen sich sowohl durch eine stark ausgeprägte systemische als auch Kontakt-Wirkung gegen saugende Insekten, insbesondere gegen saugende Insekten der Ordnung Homoptera und vor allem gegen Insekten der Familie Aphididae (wie z. B. Aphis fabae, Aphis craccivora und Myzus persicae), welche sich mit bekannten Mitteln nur schwierig bekämpfen lassen, auszeichnen.

Heben ihrer Wirkung gegenüber Mückenlarven können Verbindungen der Formel I auch zur Bekämpfung von pflanzenschädigenden Frassinsekten in Zier- und Nutzpflanzungen, insbesondere in Baumwoll- und Reiskulturen (z. B. Anthonomus grandis, Spodoptera littoralis, Heliothis virescens und Nilaparvata lugens), sowie in Obst- und Gemüsekulturen (z. B. Laspeyresia pomonella, Leptinotarsa decemlineata und Epilachna varivestis) eingesetzt werden. Die Verbindungen der Formel I zeichnen sich durch eine gute Wirkung gegen larvale Insektenstadien und Nymphen aus; zu erwähnen ist ferner ihre breite ovizide und ovolarvizide Wirkung.

Werden Verbindungen der Formel I von adulten Insekten-Stadien mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen. Weiterhin können die Verbindungen der Formel I erfolgreich gegen pflanzenschädigende Zikaden, wie Laodelphax striatellus sowie Nilaparvata lugens, und Chilo suppressalis, speziell in Reis-Kulturen, eingesetzt werden.

Die Verbindungen der Formel I können zur Bekämpfung von Ektoparasiten, wie Lucilia sericata und Zecken wie Boophilus microplus, an Haus- und Nutztieren eingesetzt werden, z. B. durch Tier-, Stall- und Weidebehandlung.

Die Wirkung der Verbindungen der Formel I sowie der sie enthaltenden Mittel lässt sich durch Zusatz von anderen Insektiziden und/oder Akariziden wesentlich verbreitern und an gegebene Umstände anpassen. Als Zusätze kommen z. B. folgende Wirkstoffe in Betracht: organische Phosphorverbindungen, Nitrophenole und Derivate, Formamidine, Harnstoffe, Carbamate, chlorierte Kohlenwasserstoffe und Bacillus thuringiensis-Präparate.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die gute insektizide Wirkung der Verbindungen der Formel I entspricht einer Abtötungsrate (Mortalität) von mindestens 50 - 60 % der erwähnten Schadinsekten.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polaren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die den Wirkstoff der Formel I bzw. Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Äther und Ester, wie Äthanol, Äthylenglykol, Äthylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Stoffe, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur, wie insbesondere Dolomit oder zerkleinerte Pflanzenrückstände verwendet werden.

Als oberflächenaktive Verbindungen kommen je nach der Art des zu formulierenden Wirkstoffs der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z. B. die Na- oder K-Salze oder Öl- oder Stearinsäure, oder von natürlichen Fettsäuregemischen, die z. B. aus Kokosnuss- oder Talgöl gewonnen werden können. Ferner sind auch die Fettsäure-methyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgeminen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit etwa 8 - 22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z. B. Salze des

Phosphorsäureesters eines p-Nonylphenol-(4-14)-Äthylenoxid-Adduktes und Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weitere geeignete nicht ionische Tenside sind die wasserlöslichen 20 bis 250 Äthylenglykoläthergruppen und 10 bis 100 Propylenglykoläthergruppen enthaltendenden Polyäthylenoxid-Addukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC
Publishing Corp., Ridgewood, New Jersey, 1982.
Dr. Helmut Stache: "Tensid Taschenbuch"
Carl Hanser Verlag München/Wien 1981.

Die pestiziden Zubereitungen enthalten in der Regel 0,1 bis 99 %, insbesondere 0,1 bis 95 %, Wirkstoff der Formel I bzw. Ia oder Kombinationen dieser Wirkstoffe mit andern Insektiziden oder Akariziden, 1 bis 99,9 % eines festen oder flüssigen Zusatzstoffes und 0 bis 25 %, insbesondere, 0,1 bis 20 %, eines Tensides. Während als Handelsware eher konzentrierte Mittel bevorzugt werden, verwendet der Endverbraucher in der Regel Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Formulierungsbeispiel für flüssige Wirkstoffe der Formel I oder Kombinationen dieser Wirkstoffe mit andern Insektiziden oder Akariziden (% = Gewichtsprozent)**

| 1. Emulsions-Konzentrate | a) | b) | c) |
|---|---|---|---|
| Wirkstoff resp. Wirkstoffkombination | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff resp. Wirkstoffkombination | 80 % | 10 % | 5 % | 95 % |
| Äthylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160 - 190° C) | - | - | 94 % | - |

Die Lösungen sind zur Anwendung in Form kleinster Tropfen geeignet.

| 3. Granulate | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff oder die Wirkstoffkombination wird in Methylenchlorid gelöst, auf den Träger aufgesprüht und das Lösungsmittel anschliessend im Vakuum abgedampft.

4. Stäubemittel

| | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit den Wirkstoff oder der Wirkstoffkombination erhält man gebrauchsfertige Stäubemittel.

**Formulierungsbeispiele für feste Wirkstoffe der Formel I resp. Kombinationen dieser Wirkstoffe mit andern Insektiziden oder Akariziden (% = Gewichtsprozent)**

5. Spritzpulver

| | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff oder Wirkstoffkombination | 10 % |
| Octylphenolpolyäthylenglykoläther (45 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

7. Stäubemittel

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

8. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

9. Umhüllungs-Granulat

| | |
|---|---|
| Wirkstoff oder Wirkstoffkobmination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff oder Wirkstoffkombination | 40 % |
| Äthylenglykol | 10 % |
| Nonylphenolpolyäthylenglykoläther (15 Mol AeO | 6 % |
| Na-Ligninsulfonat | 10 % |
| Carboxymethylcellulose | 1 % |
| 37 %-ige wässrige Formaldehyd-Lösung | 0,2 % |
| Silikonöl in Form einer 75 %-igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Beispiel 1:** a) Herstellung der Verbindung Nr. 1 der Formel

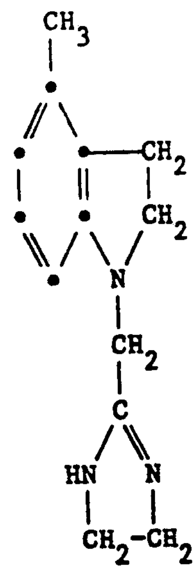

In einer zweimolaren Lösung von 45,7 g $(C_2H_5)_3$ Al in abs. Toluol werden bei 5°C unter Argon 24,0 g Äthylendiamin zugetropft. Nach der Beendigung der Gasentwicklung werden 41,0 g der Verbindung der Formel

FIG00/20

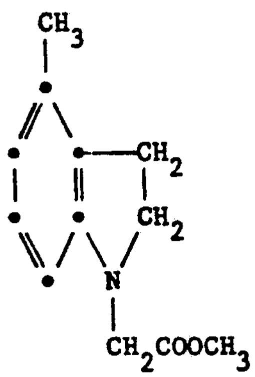

, gelöst in 40 ml Toluol, zugetropft und 3 Stunden bei 100° C gerührt.

FIG00/30

Anschliessend wird bei 30° C unter Argon vorsichtig eine Mischung von 85 ml Wasser, 280 ml Methanol und 280 ml Dichlormethan zugetropft und unter energischem Rühren 15 Minuten auf 90° C aufgeheizt.

Das Reaktionsgemisch wird auf 20° C abgekühlt, filtriert, mit 1000 ml Äthylacetat versetzt, auf dem Dampfbad 15 Minuten am Rückfluss gehalten, filtriert und unter Vakuum eingedampft. In die Lösung der rohen Base in 400 ml Dichlormethan/Tetrachlorkohlenstoff 1 : 4 leitet man bei 10°C bis zur Sättigung trockene Bromwasserstoffsäure ein. Nach dem Eindampfen und Umkristallisieren aus Wasser erhält man das Hydrobromid der Titelverbindung mit einem Schmelzpunkt von 225 - 226° C.

Zur Freisetzung der Base löst man das Salz in gesättigter Bikarbonatlösung und extrahiert 15 Stunden lang mit Dichlormethan.

Die organische Phase wird getrocknet ($Na_2SO_4$) und unter Vakuum eingedempft. Nach dem Umkristallisieren

aus Äther/Hexan 1 : 1 erhält man die Titelverbindung mit einem Schmelzpunkt von 108 - 110°C.

Auf analoge Weise werden auch folgende Verbindungen hergestellt:

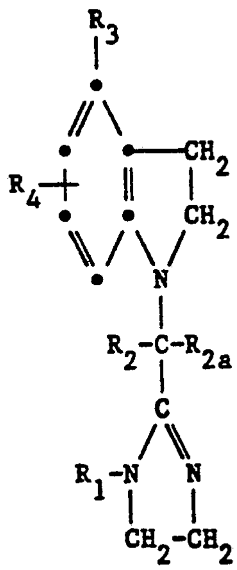

[•X]

| Nr. | $R_1$ | $R_2$ | $R_{2a}$ | $R_3$ | $R_4$ | [•X] | Physikalische Daten |
|---|---|---|---|---|---|---|---|
| 2 | H | H | H | Cl | H | •HCl | Smp.: 225°C dec. |
| 3 | H | H | H | Cl | H | | Smp.: 110-112°C |
| 5 | H | H | H | $CH_3$ | H | •HBr | Smp.: 225-226°C |
| 6 | H | H | H | H | H | •HBr | Smp.: 230°C |
| 7 | H | H | H | H | 7-Cl | •HBr | Smp.: 250°C |
| 8 | H | H | H | $CH_3$ | H | •HCl | Smp.: 200°C |
| 9 | H | H | H | H | 7-$CH_3$ | •2HBr | Smp.: 172-175°C |
| 10 | H | H | H | H | 7-Cl | | Smp.: 100°C |
| 11 | H | H | H | Cl | H | •HBr | Smp. 181-185°C |
| 12 | H | H | H | H | 7-$CH_3$ | | Smp.: 100-102°C |
| 13 | H | H | H | $CH_3$ | H | 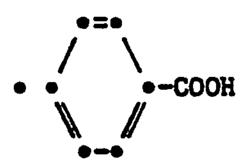 | Smp.: 145°C |

| Nr. | $R_1$ | $R_2$ | $R_{2a}$ | $R_3$ | $R_4$ | [•X] | Physikalische Daten |
|---|---|---|---|---|---|---|---|
| 14 | H | H | H | $CH_3$ | H | $\bullet CF_3COOH$ | Smp.: 155°C |
| 15 | H | H | H | $CH_3$ | H | 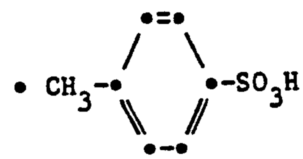 | Smp.: 160-161°C |
| 16 | H | H | H | Cl | H | 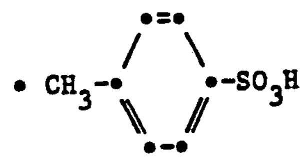 | Smp.: 174-176°C |
| 17 | H | H | H | Cl | H | •HOOC-COOH | Smp.: 224°C |
| 18 | H | H | H | Cl | H | 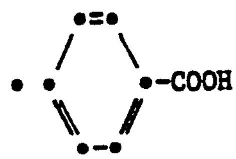 | Smp.:135-137°C |
| 19 | H | H | H | Cl | H | $\bullet CF_3COOH$ | Smp.: 147-149°C |
| 20 | H | H | H | H | 5-Cl | | |
| 21 | H | H | H | $CH_3$ | 7-$CH_3$ | | |
| 22 | H | H | H | H | 6-$OCH_3$ | | |
| 23 | H | H | H | $CF_3$ | H | | |
| 24 | H | H | H | $OCH_3$ | H | | |
| 25 | H | H | H | F | H | | |
| 26 | H | H | H | Br | H | | |

b) Herstellung der Verbindung Nr. 27 der Formel

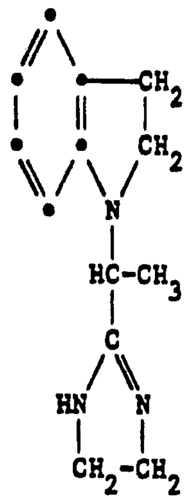

Eine Lösung von 0,02 Mol der Verbindung der Formel

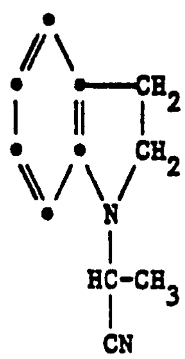

0,04 Mol Äthylendiamin und 3 - 5 Tropfen Schwefelkohlenstoff werden bei einer Temperatur von 110 - 120°C energisch gerührt. Das Reaktionsgemisch wird auf Eis gegeben und mit Dichlormethan extrahiert. Die organische Phase wird dreimal mit Wasser und einmal mit gesättigter Kochsalzlösung gewaschen. Nach dem Trocknen über Natriumsulfat, dem Eindampfen und Chromatographieren (Kieselgel; Eluiermittel Methylalkohol/Ammoniak 20 : 1) erhält man die Titelverbindung, welche im Massenspektrum Ausschläge bei 215, 144, 130, 117 und 97 $\frac{m}{e}$ aufweist.

Auf analoge Weise werden auch folgende Verbindungen hergestellt:

Nr.28 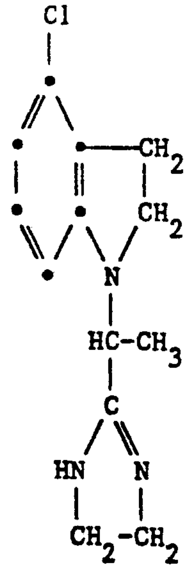

Nr.29 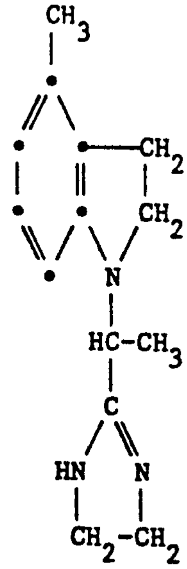

**Beispiel 2:** Insektizide systemische Wirkung: Aphis craccivora

Bewurzelte Bohnenpflanzen werden in Töpfe, welche 600 ccm Erde enthalten, verpflanzt. Anschliessend werden 50 ml einer Versuchslösung, enthaltend 25 ppm bzw 5 ppm der zu prüfenden Verbindung, direkt auf die Erde gegossen.

Nach 24 Stunden werden auf die oberirdischen Pflanzenteile Blattläuse (Aphis craccivora) gesetzt und die Pflanzen mit eine unten zugeschnürten Plastikzylinder überstülpt, um die Läuse vor einer eventuellen Kontakt- oder Gaswirkung der Testsubstanz zu schützen.

Die Auswertung der erzielten Abtötung erfolgt 48 Stunden nach Versuchsbeginn. Pro Konzentrationsdosis Testsubstanz werden zwei Pflanzen, je eine in einem separaten Topf, verwendet. Der Versuch wird bei 25°C und 70 % relativer Luftfeuchtigkeit durchgeführt.

Die Verbindungen gemäss Beispiel 1 haben die in der folgenden Tabelle angegebenen Wirkungen gegen Aphis craccivora.

**Biologische Versuchsergebnisse:**

In der folgenden Tabelle sind Versuchsergebnisse auf der Basis des vorstehenden Beispiele aufgeführt, und zwar mit folgendem Bewertungsindex in bezug auf die prozentuale Abtötung der Schädlinge:

A: 70-100 % Abtötung bei 5 ppm Wirkstoffkonzentration
B: 70-100 % Abtötung bei 25 ppm Wirkstoffkonzentration

| Verbindung | Wirksamkeit gegen Aphis craccivora |
|---|---|
| 1 | B |
| 2 | B |
| 3 | B |
| 5 | B |
| 6 | B |
| 7 | B |
| 8 | A |
| 9 | A |
| 10 | B |
| 11 | B |
| 12 | B |
| 13 | B |
| 14 | B |
| 15 | B |
| 16 | B |

**Beispiel 3:** Akarizide Wirkung: Boophilus microplus (Larven)

Je 20 sensible resp. OP-resistente Zeckenlarven (die Resistenz bezieht sich auf die Verträglichkeit von Diazinon) werden in ein Glasröhrchen gezählt und für 1 bis 2 Minuten in 2 ml einer wässrigen Emulsion aus einer Verdünnungsreihe mit je 100, 10, 1 oder 0,1 ppm Testsubstanz getaucht. Das Röhrchen wird dann mit einem genormten Wattebausch verschlossen und auf den Kopf gestellt, damit die Wirkstoffemulsion von der Watte aufgenommen werden kann.

Die Verbindungen gemäss Beispiel 1 haben die in der folgenden Tabelle angegebene Wirkung gegen Boophilus microplus.

**Biologische Versuchsergebnisse:**

In der folgenden Tabelle sind Versuchsergebnisse auf der Basis des vorstehenden Beispiels aufgeführt, und zwar mit folgendem Bewertungsindex in bezug auf die prozentuale Abtötung der Larven von Boophilus microplus

A: 70-100 % Abtötung bei 0,1 ppm Wirkstoffkonzentration
B: 70-100 % Abtötung bei 1 ppm Wirkstoffkonzentration
C: 70-100 % Abtötung bei 10 ppm Wirkstoffkonzentration
D: 70-100 % Abtötung bei 100 ppm Wirkstoffkonzentration

| Verbindung Nr. | Wirksamkeit gegen Boophilus microplus sensible Larven | OP-resistente Larven |
|---|---|---|
| 1 | A | B |
| 2 | B | C |
| 3 | A | B |

**Beispiel 4:** Insektizide Frassgift-Wirkung: Nilaparvata lugens

Reispflanzen werden mit einer Versuchslösung, enthaltend 50 ppm der zu prüfenden Verbindung, besprüht. Nach dem Antrocknen des Belages werden die Pflanzen mit Larven von Nilaparvata lugens (L3-Stadium) besetzt. Man verwendet pro Versuchsverbindung und pro Test-Spezies zwei Pflanzen. Die Auswertung der erzielten Abtötungsrate erfolgt nach 24 Stunden. Der Versuch wird bei 22°C und 60 % relativer Luftfeuchtigkeit durchgeführt.

Verbindungen gemäss Beispiel 1 zeigen im obigen Test 100 % Wirkung gegenüber Nilaparvata lugens-Larven.

**Patentansprüche**

1. Eine Verbindung der Formel I

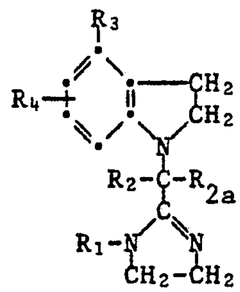

(I)

oder deren Salze,
worin,
$R_1$ Wasserstoff oder $C_1$-$C_{12}$-Alkyl,
$R_2$ und $R_{2a}$ unabhängig voneinander Wasserstoff, $C_1$-$C_4$-Alkyl oder zusammen $C_2$-$C_4$-Alkylen,
$R_3$ Wasserstoff, Halogen, $C_1$-$C_3$-Alkoxy oder gegebenenfalls durch Halogen substituiertes $C_1$-$C_4$-Alkyl und
$R_4$ Halogen, $C_1$-$C_3$-Alkoxy oder gegebenenfalls durch Halogen substituiertes $C_1$-$C_4$-Alkyl oder
$R_3$ Halogen, $C_1$-$C_3$-Alkoxy oder gegebenenfalls durch Halogen substituiertes $C_1$-$C_4$-Alkyl und
$R_4$ Wasserstoff, Halogen, $C_1$-$C_3$-Alkoxy oder gegebenenfalls durch Halogen substituiertes $C_1$-$C_4$-Alkyl
bedeuten.

2. Eine Verbindung der Formel I gemäss Anspruch 1, worin
$R_1$ und $R_{2a}$ Wasserstoff,
$R_2$ Wasserstoff oder $C_1$-$C_4$-Alkyl,
$R_3$ Halogen, $C_1$-$C_3$-Alkoxy oder gegebenenfalls durch Fluor substituiertes $C_1$-$C_4$-Alkyl und
$R_4$ Wasserstoff, Halogen oder $C_1$-$C_4$-Alkyl bedeuten.

3. Eine Verbindung der Formel I gemäss Anspruch 2, worin
$R_1$, $R_2$, $R_{2a}$ und $R_4$ Wasserstoff und
$R_3$ Halogen, $C_1$-$C_3$-Alkoxy oder gegebenenfalls durch Fluor substituiertes $C_1$-$C_4$-Alkyl bedeuten.

4. Die Verbindung gemäss Anspruch 3 der Formel

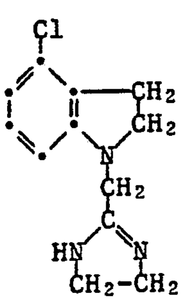

• HCl

5. Die Verbindung gemäss Anspruch 3 der Formel

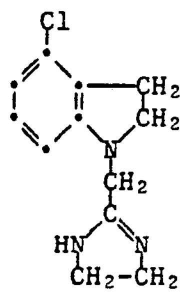

.

6. Die Verbindung gemäss Anspruch 3 der Formel

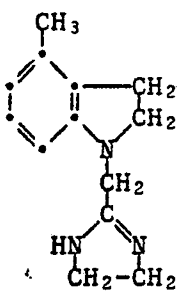

.

7. Die Verbindung gemäss Anspruch 3 der Formel

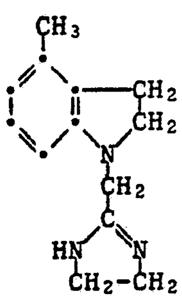

• HBr .

8. Die Verbindung gemäss Anspruch 1 der Formel

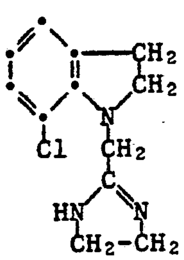

• HBr .

9. Die Verbindung gemäss Anspruch 3 der Formel

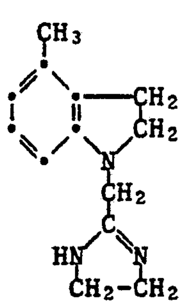

• HCl .

10. Die Verbindung gemäss Anspruch 1 der Formel

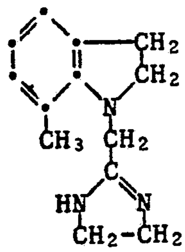

• 2HBr .

11. Die Verbindung gemäss Anspruch 1 der Formel

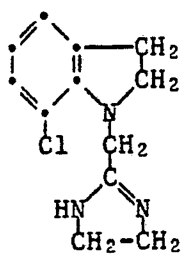

.

12. Die Verbindung gemäss Anspruch 3 der Formel

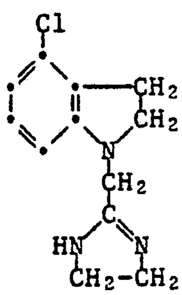

•HBr .

13. Die Verbindung gemäss Anspruch 1 der Formel

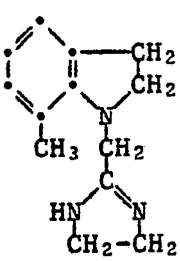

.

14. Die Verbindung gemäss Anspruch 3 der Formel

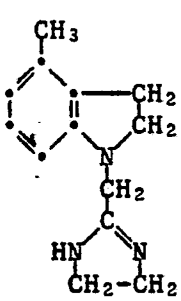

•

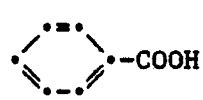

.

15. Die Verbindung gemäss Anspruch 3 der Formel

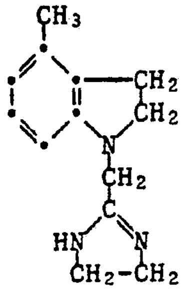

• $CF_3COOH$ .

16. Die Verbindung gemäss Anspruch 3 der Formel

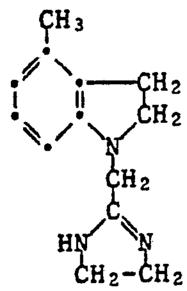

•

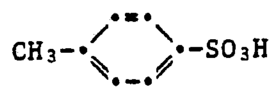

.

17. Die Verbindung gemäss Anspruch 3 der Formel

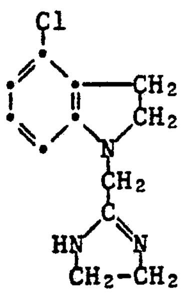

•

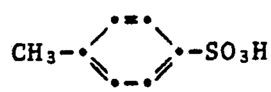

.

18. Die Verbindung gemäss Anspruch 3 der Formel

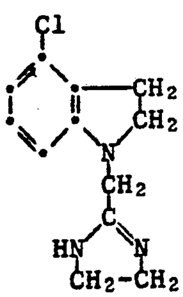

• HOOC–COOH .

19. Die Verbindung gemäss Anspruch 3 der Formel

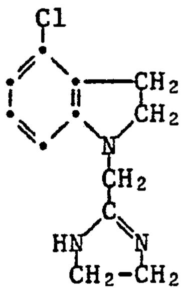 • 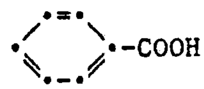 .

20. Die Verbindung gemäss Anspruch 3 der Formel

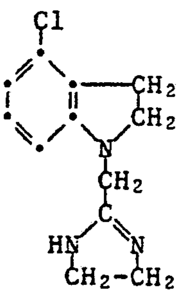 • $CF_3COOH$ .

21. Die Verbindung gemäss Anspruch 1 der Formel

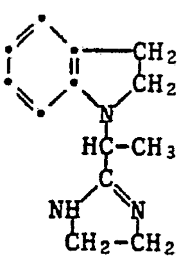 .

22. Ein Mittel zur Bekämpfung von Insekten und Vertretern der Ordnung Akarina, welches als aktive Komponente eine Verbindung der Formel I, gemäss Anspruch 1

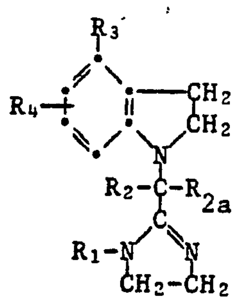

(I)

oder deren Salze, zusammen mit geeigneten Trägern und/oder andern Zuschlagstoffen enthält.

23. Ein Mittel gemäss Anspruch 22 enthaltend als aktive Komponente eine Verbindung der Formel I, worin $R_1$ und $R_{2a}$ Wasserstoff, $R_2$ Wasserstoff oder $C_1$-$C_4$-Alkyl, $R_3$ Halogen, $C_1$-$C_3$-Alkoxy oder gegebenenfalls durch Fluor substituiertes $C_1$-$C_4$-Alkyl und $R_4$ Wasserstoff, Halogen oder $C_1$-$C_4$-Alkyl bedeuten.

24. Ein Mittel gemäss Anspruch 23 enthaltend als aktive Komponente eine Verbindung der Formel I, worin $R_1$, $R_2$, $R_{2a}$ und $R_4$ Wasserstoff und
$R_3$ Halogen, $C_1$-$C_3$-Alkoxy oder gegebenenfalls durch Fluor substituiertes $C_1$-$C_4$-Alkyl bedeuten.

25. Die Verwendung mit Ausnahme der therapeutischen Verwendung eines Mittels gemäss Anspruch 22 zur Bekämpfung von verschiedenartigen Schädlingen an Tiere und Pflanzen.

26. Die Verwendung gemäss Anspruch 25 zur Bekämpfung von Insekten und Vertretern der Ordnung Akarina.

27. Verfahren zur Herstellung von Verbindungen gemäss Anspruch 1 der Formel I, dadurch gekennzeichnet, daß man eine Verbindung der Formel

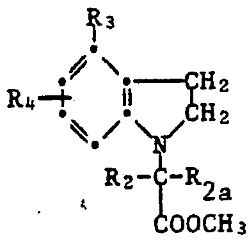

oder

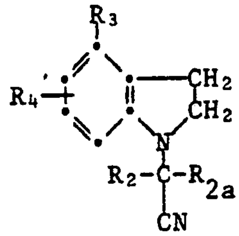

in Gegenwart von Triäthylaluminium resp. Schwefelkohlenstoff mit einer Verbindung der Formel

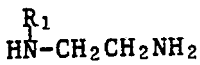

umsetzt, worin $R_1$, bis $R_4$ die im Anspruch 1 angegebene Bedeutung haben.

## Claims

1. A compound of the formula I

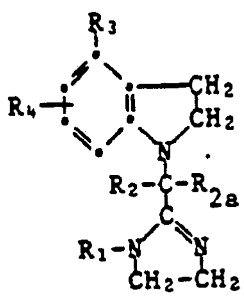

(I)

or salts thereof
wherein
$R_1$ is hydrogen or $C_1$-$C_{12}$-alkyl,
$R_2$ and $R_{2a}$ independently of one another are each hydrogen or $C_1$-$C_4$-alkyl, or together are $C_2$-$C_4$-alkylene, and
$R_3$ is hydrogen, halogen or $C_1$-$C_3$-alkoxy, or $C_1$-$C_4$-alkyl which is unsubstituted or substituted by halogen, and
$R_4$ is halogen or $C_1$-$C_3$-alkoxy, or $C_1$-$C_4$-alkyl which is unsubstituted or substituted by halogen,
or
$R_3$ is halogen or $C_1$-$C_3$-alkoxy, or $C_1$-$C_4$-alkyl which is unsubstituted or substituted by halogen, and
$R_4$ is hydrogen, halogen or $C_1$-$C_3$-alkoxy, or $C_1$-$C_4$-alkyl which is unsubstituted or substituted by halogen.

2. A compound of the formula I according to claim 1, wherein
$R_1$ and $R_{2a}$ are hydrogen,
$R_2$ is hydrogen or $C_1$-$C_4$-alkyl,
$R_3$ is halogen or $C_1$-$C_3$-alkoxy, or $C_1$-$C_4$-alkyl which is unsubstituted or substituted by fluorine, and
$R_4$ is hydrogen, halogen or $C_1$-$C_4$-alkyl.

3. A compound of the formula I according to claim 2, wherein
$R_1$, $R_2$, $R_{2a}$ and $R_4$ are hydrogen, and
$R_3$ is halogen or $C_1$-$C_3$-alkoxy, or $C_1$-$C_4$-alkyl which is unsubstituted or substituted by fluorine.

4. The compound according to claim 3 of the formula

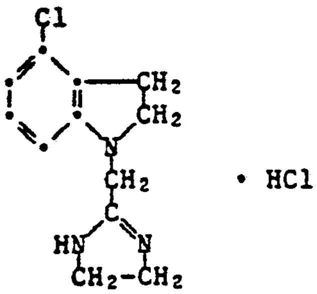

.

5. The compound according to claim 3 of the formula

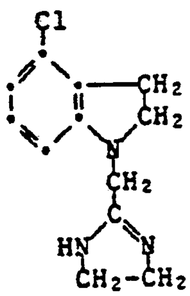

.

6. The compound according to claim 3 of the formula

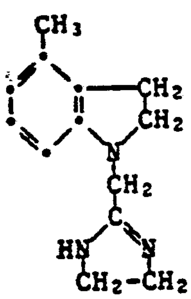

.

7. The compound according to claim 3 of the formula

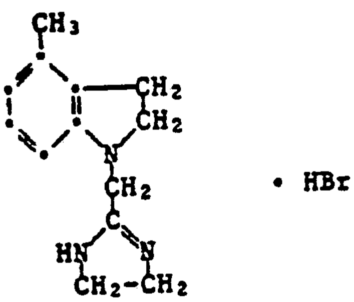

.

8. The compound according to claim 1 of the formula

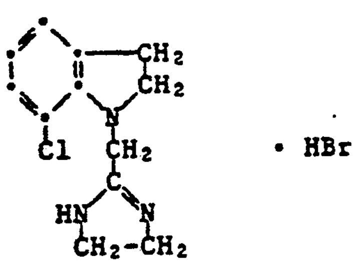

.

9. The compound according to claim 3 of the formula

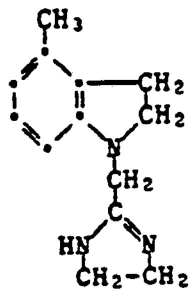

• HCl

.

10. The compound according to claim 1 of the formula

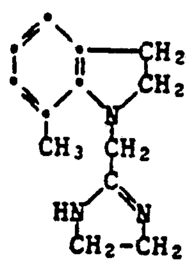

• 2HBr

.

11. The compound according to claim 1 of the formula

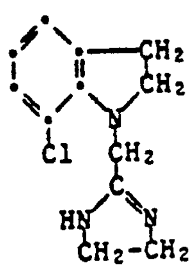

.

12. The compound according to claim 3 of the formula

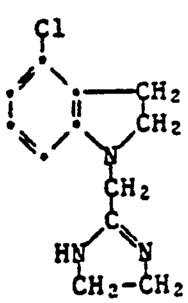

•HBr

.

13. The compound according to claim 1 of the formula

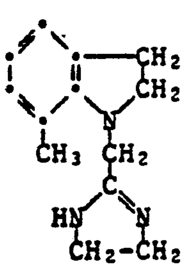

.

14. The compound according to claim 3 of the formula

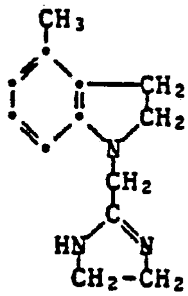 • 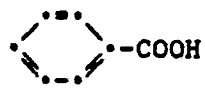 .

15. The compoundaccording to claim 3 of the formula

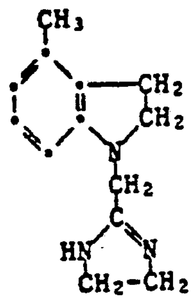 • $CF_3COOH$ .

16. The compound according to claim 3 of the formula

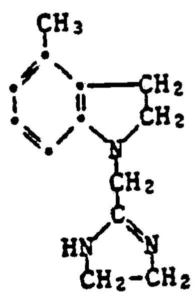 • 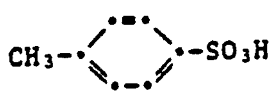 .

17. The compound according to claim 3 of the formula

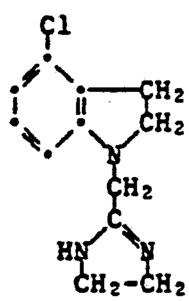 • 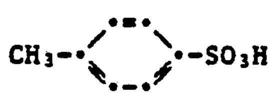 .

18. The compound according to claim 3 of the formula

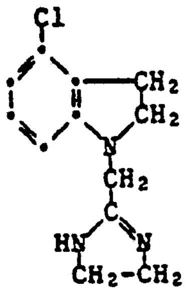

• HOOC-COOH

.

19. The compound according to claim 3 of the formula

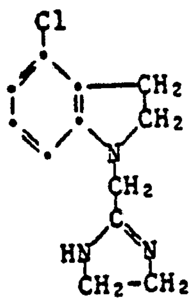

• 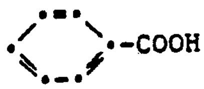

.

20. The compound according to claim 3 of the formula

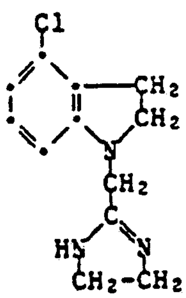

• $CF_3COOH$

.

21. The compound according to claim 1 of the formula

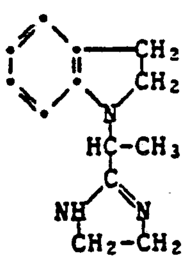

.

22. A composition for controlling insects, and members of the order Acarina, which composition contains, as active ingredient, a compound of the formula I, according to claim 1

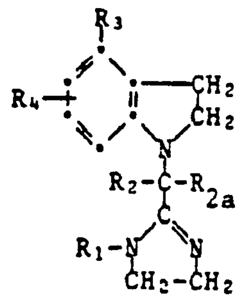

(I)

or salts thereof, together with suitable carriers and/or other additives.

23. A composition according to claim 22, which contains, as active ingredient, a compound of the formula I wherein $R_1$ and $R_{2a}$ are hydrogen, $R_2$ is hydrogen or $C_1$-$C_4$-alkyl, $R_3$ is halogen or $C_1$-$C_3$-alkoxy, or $C_1$-$C_4$-alkyl which is unsubstituted or substituted by fluorine, and $R_4$ is hydrogen, halogen or $C_1$-$C_4$-alkyl.

24. A composition according to claim 23, which contains, as active ingredient, a compound of the formula I wherein

$R_1$, $R_2$, $R_{2a}$ and $R_4$ are hydrogen, and

$R_3$ is halogen or $C_1$-$C_3$-alkoxy, or $C_1$-$C_4$-alkyl which is unsubstituted or substituted by fluorine.

25. The use, with the exception of the therapeutic use, of a composition according to claim 22 for controlling various pests on animals and plants.

26. The use according to claim 25 for controlling insects, and members of the order Acarina.

27. A process for producing compounds according to claim 1 of the formula I, characterised in that a compound of the formula

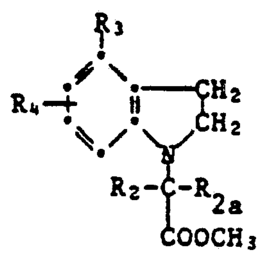

or

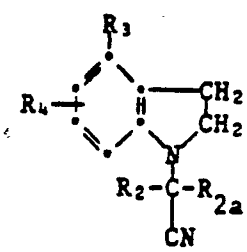

is reacted in the presence of triethylaluminium and carbon disulfide, respectively, with a compound of the formula

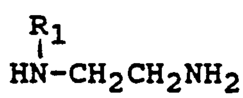

wherein $R_1$ to $R_4$ have the meanings defined in claim 1.

**Revendications**

1. Un composé de formule I

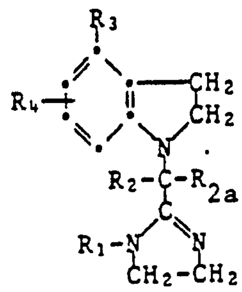

(I)

ou ses sels,
dans lesquels

$R_1$ représente l'hydrogène ou un groupe alkyle en $C_1$-$C_{12}$,

$R_2$ et $R_{2a}$ représentent chacun, indépendamment l'un de l'autre, l'hydrogène, un groupe alkyle en $C_1$-$C_4$, ou bien, ensemble, un groupe alkylène en $C_2$-$C_4$ et

$R_3$ représente l'hydrogène, un halogène, un groupe alcoxy en $C_1$-$C_3$ ou un groupe alkyle en $C_1$-$C_4$ éventuellement substitué par un halogène et

$R_4$ représente un halogène, un groupe alcoxy en $C_1$-$C_3$ ou un groupe alkyle en $C_1$-$C_4$ éventuellement substitué par un halogène, ou bien

$R_3$ représente un halogène, un groupe alcoxy en $C_1$-$C_3$ ou un groupe alkyle en $C_1$-$C_4$ éventuellement substitué par un halogène et

$R_4$ représente l'hydrogène, un halogène, un groupe alcoxy en $C_1$-$C_3$ ou un groupe alkyle en $C_1$-$C_4$ éventuellement substitué par un halogène.

2. Un composé de formule I selon la revendication 1, dans lequel

$R_1$ et $R_{2a}$ représentent l'hydrogène,

$R_2$ représente l'hydrogène ou un groupe alkyle en $C_1$-$C_4$,

$R_3$ représente un halogène, un groupe alcoxy en $C_1$-$C_3$ ou un groupe alkyle en $C_1$-$C_4$ éventuellement substitué par le fluor, et

$R_4$ représente l'hydrogène, un halogène ou un groupe alkyle en $C_1$-$C_4$.

3. Un composé de formule I selon la revendication 2, dans lequel

$R_1$, $R_2$ $R_{2a}$ et $R_4$ représentent l'hydrogène et

$R_3$ représente un halogène, un groupe alcoxy en $C_1$-$C_3$ ou un groupe alkyle en $C_1$-$C_4$ éventuellement substitué par le fluor.

4. Le composé selon la revendication 3, de formule

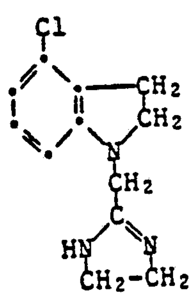

• HCl

5. Le composé selon la revendication 3, de formule

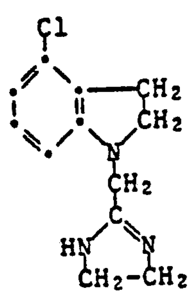

6. Le composé selon la revendication 3, de formule

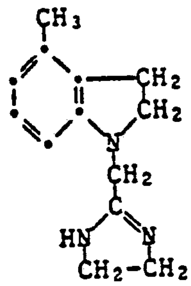

7. Le composé selon la revendication 3, de formule

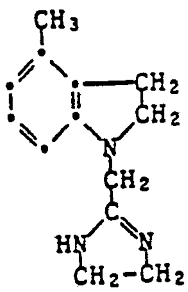

• HBr

8. Le composé selon la revendication 1, de formule

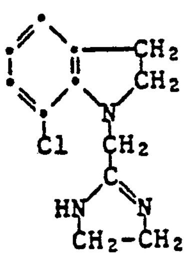

• HBr

9. Le composé selon la revendication 3, de formule

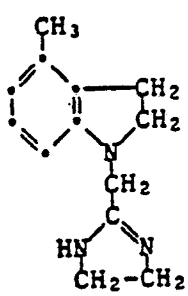

• HCl

10. Le composé selon la revendication 1, de formule

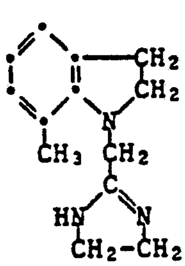

• 2HBr

11. Le composé selon la revendication 1, de formule

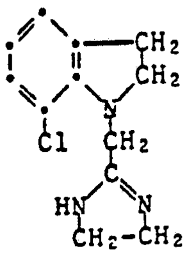

12. Le composé selon la revendication 3, de formule

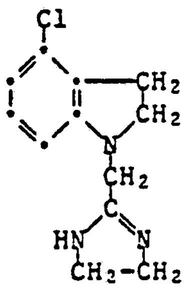

•HBr

13. Le composé selon la revendication 1, de formule

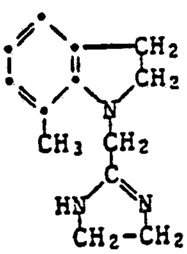

14. Le composé selon la revendication 3, de formule

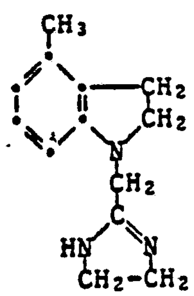

•

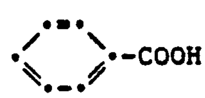

15. Le composé selon la revendication 3, de formule

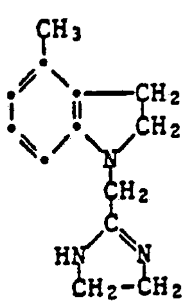

• $CF_3COOH$

16. Le composé selon la revendication 3, de formule

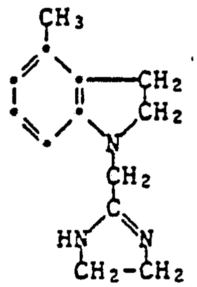 · 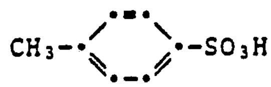

.

17. Le composé selon la revendication 3, de formule

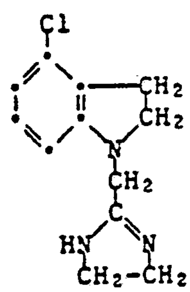 · 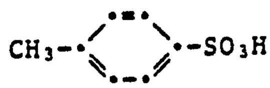

.

18. Le composé selon la revendication 3, de formule

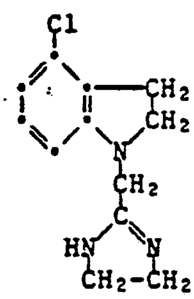 · HOOC-COOH

.

19. Le composé selon la revendication 3, de formule

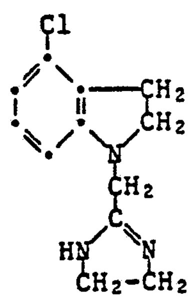 · 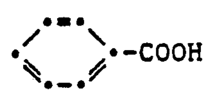

.

20. Le composé selon la revendication 3, de formule

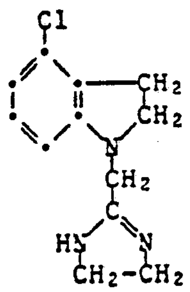

• $CF_3COOH$

.

21. Le composé selon la revendication 1, de formule

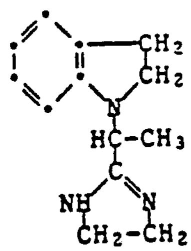

22. Un produit pour combattre les insectes et les représentants de l'ordre des acariens, qui contient en tant que composant actif un composé de formule I selon la revendication 1

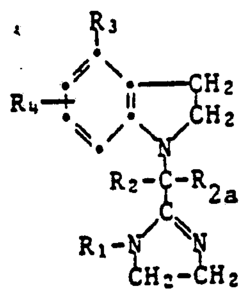

(I)

ou ses sels, avec des vehicules et/ou d'autres additifs appropriés.

23. Un produit selon la revendication 22, contenant en tant que composant actif un composé de formule I dans laquelle $R_1$ et $R_{2a}$ représentent l'hydrogène, $R_2$ représente l'hydrogène ou un groupe alkyle en $C_1$-$C_4$, $R_3$ représente un halogène, un groupe alcoxy en $C_1$-$C_3$ ou un groupe alkyle en $C_1$-$C_4$ éventuellement substitué par le fluor et $R_4$ représente l'hydrogène, un halogène ou un groupe alkyle en $C_1$-$C_4$.

24. Un produit selon la revendication 23, contenant en tant que composant actif un composé de formule I dans laquelle

$R_1$, $R_2$, $R_{2a}$ et $R_4$ représentent l'hydrogène et

$R_3$ représente un halogène, un groupe alcoxy en $C_1$-$C_3$ ou un groupe alkyle en $C_1$-$C_4$ éventuellement substitué par le fluor.

25. L'utilisation, à l'exception de l'utilisation thérapeutique, d'un produit selon la revendication 22 dans la lutte contre des parasites variés des animaux et des végétaux.

26. L'utilisation selon la revendication 25 dans la lutte contre les insectes et les représentants de l'ordre des acariens.

27. Procédé de préparation des composés de formule I selon la revendication 1, caractérisé en ce que l'on fait réagir un composé de formule

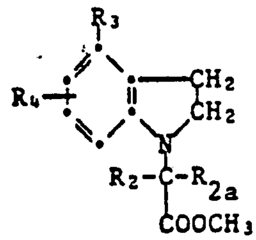 ou 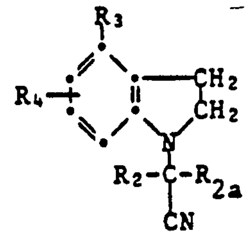

en présence respectivement de triéthyl-aluminium et de sulfure de carbone, avec un composé de formule

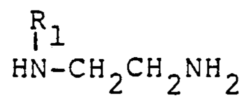

dans laquelle les symboles $R_1$ à $R_4$ ont les significations indiquées dans la revendication 1.